(19) Europäisches Patentamt
European Patent Office
Office européen des brevets

(11) Publication number: **0 223 744 B1**

(12) **EUROPEAN PATENT SPECIFICATION**

(45) Date of publication of patent specification: **11.03.92**

(51) Int. Cl.[5]: **C07D 405/04**, C07D 405/14, A61K 31/445

(21) Application number: **86830300.9**

(22) Date of filing: **20.10.86**

The file contains technical information submitted after the application was filed and not included in this specification

(54) **Flavone derivatives.**

(30) Priority: **22.10.85 IT 2257885**

(43) Date of publication of application:
**27.05.87 Bulletin 87/22**

(45) Publication of the grant of the patent:
**11.03.92 Bulletin 92/11**

(84) Designated Contracting States:
**AT BE CH DE ES FR GB GR LI LU NL SE**

(56) References cited:
**EP-A- 0 123 112**
**US-A- 2 921 070**

**DEUTSCHE APOTHEKER ZEITUNG 104, vol. 3, 1983, pages 139-146; R. MANNHOLD et al.: "Pharmakologische Differenzierung von Calcium-Antagonisten"**

**Nature 303, 535 (1983) Drug. Res. 33, 1268 (1983)**

(73) Proprietor: **RECORDATI S.A. CHEMICAL and PHARMACEUTICAL COMPANY**
**Corso S. Gottardo 54**
**CH-6830 Chiasso(CH)**

(72) Inventor: **Leonardi, Amedeo**
**Via Poliziano 16**
**Milan(IT)**
Inventor: **Pennini, Renzo**
**Via Ricciarelli, 8**
**Milan(IT)**
Inventor: **Cazzulani, Pietro**
**Viale dei Cappuccini, 27**
**Casalpusterlengo Milan(IT)**
Inventor: **Nardi, Dante**
**Via T. Gulli, 47**
**Milan(IT)**

(74) Representative: **Righetti, Giuseppe**
**Bugnion S.p.A. Via Carlo Farini, 81**
**I-20159 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention concerns some new flavone derivatives with a calcium-antagonistic activity as well as a relaxing activity on the smooth musculature (in particular on the bladder), some procedures for their preparation, and pharmaceutical compositions containing them.

From US 2921070 it is known that some esters of 3-methylflavon-8-carboxylic acid possess an excellent spasmolitic activity. Moreover it is commonly known from a lot of examples in literature, that the 1,4-dihydropyridine nucleus bearing in 3,5 position substituted estereal functions is characteristic of products exhibiting a calcium-antagonistic activity. Now it has been found that combining the 3-methylflavonic moiety of the active molecules described in the above mentioned US 2921070 with such 1,4-dihydropyridine nucleus, new compounds having a calcium-antagonistic activity and/or an activity relaxing the smooth musculature (in particular the bladder) are obtained.

In DE 3311005 some derivatives of 1,4-dihydropyridine substituted with chromone or thiochromone are described, but in this patent all the products are esters, being able to bear at the 5 position of the dihydropyridinic ring only hydrogen, or the nitro, cyano, halogen, alkyl, fluoroalkyl and hydroxycarbonyl groups. Furthermore they are claimed to be suitable as cardiotonic agents, to improve heart contractility, as anti-hypotonic agents, to lower blood sugar, for detumescing mucous membranes and to influence salt and liquid balance.

Object of the present invention are compounds of general formula

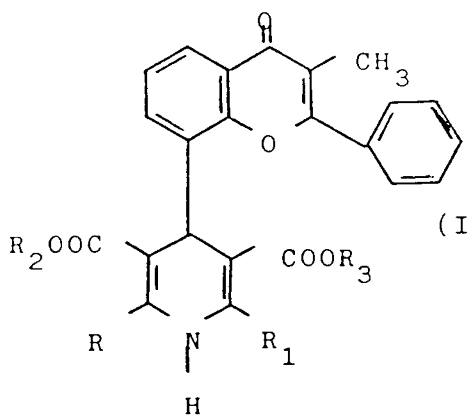

(I)

wherein R and $R_1$ can be equal or different and are alkyl containing from 1 to 4 carbon atoms, hydroxyalkyl having 1-4 carbon atoms, formylalkyl or cyanoalkyl, $R_2$ and $R_3$ can be equal or different and are straight or branched alkyl containing from 1 to 6 carbon atoms, straight or branched alkenyl containing from 2 to 6 carbon atoms, alkynyl containing from 2 to 6 carbon atoms, cycloalkyl having 5-7 carbon atoms, aralkyl, phenyl, cyanoalkyl, haloalkyl, mono- or poly-hydroxyalkyl, alkoxyalkyl, alkylthioalyyl, alkylsulfonylalkyl, acyloxyalkyl, acylalkyl, $R_4R_5$N-alkyl straight or branched having 2-6 carbon atoms, where $R_4$ and $R_5$ are equal or different and are hydrogen, alkyl, cycloalkyl, aralkyl, phenyl, 3,3-diphenylpropyl, their optical isomers and diastereoisomers, and pharmaceutically acceptable salts thereof.

The compounds of the invention having formula (I) may be obtained:

a) reacting an aldheyde of formula

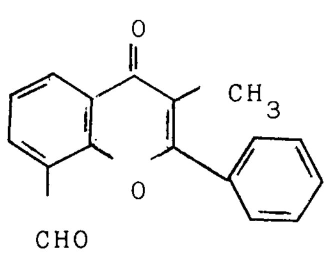

(II)

(hereinafter identified as II) with a ketoester of formula

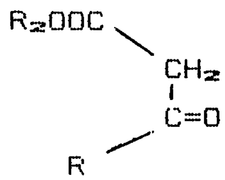

(III)

and with an enamine of formula

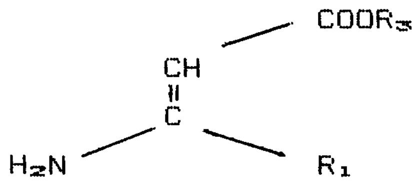

(IV)

wherein R, $R_1$, $R_2$, and $R_3$ have the above mentioned meanings and R, $R_1$ can be transformed by means of known procedures in formylalkyl, hydroxyalkyl, or cyanoalkyl groups, or alternatively:

b) treating (II) with a compound of formula

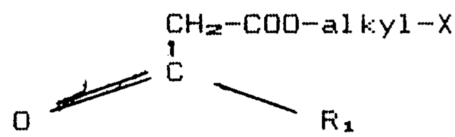

(V)

wherein for $R_1$ is worth that said in a) and $_X$ is a halogen atom, and making to react the arylidenederivative thus obtained with an enamine of formula

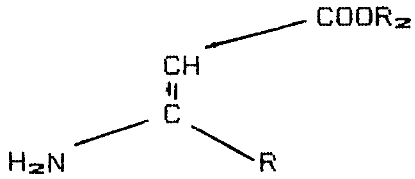

(VI)

wherein R, and $R_2$ have the above mentioned meanings, and at last optionally adding an amine of formula $R_4R_5NH$, wherein $R_4$ and $R_5$ are as defined above or alternatively

c) reacting the aldehyde (II) with a ketoester of formula (III) and then adding to the obtained arylidenederivative an enamine of formula

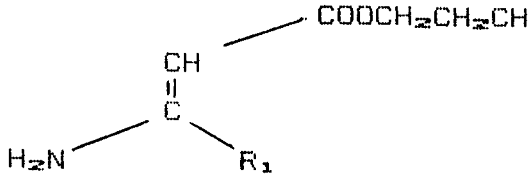

(VII)

wherein R, $R_1$, and $R_2$ have the above mentioned meanings or is worth what said in a), further hydrolizing the 1,4-dihydropyridinic cyanoethylester thus obtained to give the acid of formula

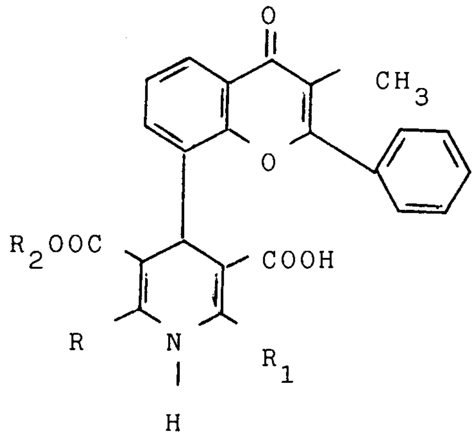

(VIII)

and then esterifying (VIII) by means of known procedures with a derivative of formula $R_3OH$ or $R_3X$, where X is a halogen atom and $R_3$ has the above described meaning.

The reaction of route a) is generally performed in a lower alcohol, at the reflux temperature of the employed alcohol and for a period of 2-5 hours.

Ethanol is the preferred alcohol. After cooling and standing, the precipitated product is isolated in the usual manner and optionally purified by recrystallization.

The reaction between (II) and (V) of the route b) is generally performed in a chlorinated hydrocarbon, preferably chloroform, whereas the further addition of (VI) in a lower alcohol, preferably isopropanol. The optional further reaction with the amine of formula $R_4R_5NH$ is performed in dimethylformamide, under stirring, at the temperature of about 100 °C. The reaction of route c) is performed for the first step in chloroform, the following addition of the enamine (VII) in alcohol (isopropanol), then the hydrolysis of the cyanoethylester in dimethoxyethane and at last the esterification of the acid thus obtained in dimethylformamide.

The transformation of R and $R_1$ in formylalkyl group is performed by cyclization of a compound of formula (VII) or (III) in which R or $R_1$ is geminal dialkoxyalkyl and then by hydrolysis, with hydrochloric acid in acetone, of the obtained 1,4-dihydropyridinic ring of formula (I), having in R or $R_1$ the above mentioned dimethoxyalkyl group.

The formalkyl group can be reduced to the corresponding hydroxyalkyl group using sodium borohydride in ethanol, or alternatively can be converted in the corresponding cyanoalkyl group, by means of hydroxylamine and acetic anhydride.

More details about all the preparation routes of the compounds of formula (I) are deducible from the Examples. All the intermediates of formula (III)-(VIII) are known or can be prepared following known procedures.

The salts according to the invention may be prepared from the basic esters obtained from the processes described above according to conventional methods such as addition of an acid to the free base dissolved in a suitable solvent. Suitable acids include hydrogen halides, phosphoric acid, nitric acid, alkylsulfonic acids, arylsulfonic acids, monofunctional and bifunctional carboxylic acids, hydroxycarboxylic acids and 1,5-naphtalenedisulfonic acid, isolation and purification may be effected conventionally.

The new compounds of general formula (I) object of the present invention possess an excellent calcium-antagonistic activity and/or a relaxing activity on the smooth musculature (in particular on the bladder) and moreover they are characterized by a low toxicity.

Therefore are further object of the present invention pharmaceutical compositions containing as active constituent a compound of formula (I) together with the usual supports and diluents. The formulations can appear in form of tablets, capsules, pills, granulated, syrups, emulsions, suspensions and solutions, and are prepared in usual manner mixing the active substances with solvents and/or supports, optionally adding emulsifying agents and/or dispersive agents, whenever water is used as diluent, also organic solvents can be used as adjuvants. The administration occurs in usual manner, preferably per oral or parenteral route. In the case of oral employment, the pharmaceutical forms suitable for this purpose can contain also additives as well as various supplementary substances, like starch, gelatine and the like. In the case of liquid forms, compatible colorants, or taste-correcting substances can be added. For the parenteral administration, the solutions of the active principle are added with usual liquid eccipients well known by the skilled in the art.

The new compounds inhibit the receptor binding of $^3$H-nitrendipine, thus demonstrating the calcium-antagonistic activity. They augment the bladder capacity and reduce the micturition pressure and the contractility of the detrusor muscle besides having a very low toxicity. From tests performed on the rat, it has been proved that in order to have an optimal activity relaxing the bladder smooth musculature it is generally suitable to administer these compounds per oral route.

The $DL_{50}$ of the new compounds was determined in mice both i.p. and p.o., following the method described by C.S. Weil (Biometrics, 8, 249, 1952). As example, some of the results obtained are reported in Table 1.

Table 1

| $LD_{50}$ mg/kg (mice) | | |
|---|---|---|
| active compound | i.p. | p.o. |
| i | 889 | 3000 |
| iii | 1000 | 3000 |
| iv | 222 | 3000 |
| v | 959 | 3000 |
| vi | 1000 | 3000 |
| viii | 1000 | 3000 |
| xii | 597 | 3000 |

The activity on calcium-antagonistic binding site was detected "in vitro" by displacement of $^3$H-nitrendipine, according to the method of Bolger et al. (J. Pharm. Exp. Ther., 225, 291, 1983).

For the binding essays, rat brain membranes were used, the reaction mixture (2 ml) was incubated for 60' at 25° C together with $^3$H-nitrendipine (0.45 nM) and various concentrations of the tested compound. $IC_{50}$'s values were determined from inhibition curves derived from the binding of the $^3$H-nitrendipine to its respective binding site in presence of the antagonist compound, and calculated by non linear fitting.

As example, some of the results obtained are reported in Table 2.

Table 2

| Activity on calcium-antagonistic binding site (rat) | |
|---|---|
| active compound | $IC_{50}$ (nM) |
| i | $5.55\ 10^{-9}$ |
| iii | $8.81\ 10^{-9}$ |
| iv | $3.03\ 10^{-7}$ |
| v | $8.38\ 10^{-9}$ |
| vi | $1.30\ 10^{-7}$ |
| viii | $3.06\ 10^{-9}$ |
| x | $1.35\ 10^{-8}$ |
| xi | $5.45\ 10^{-8}$ |
| xii | $3.19\ 10^{-8}$ |
| xiv | $1.46\ 10^{-8}$ |
| xvi | $3.96\ 10^{-9}$ |
| xviii | $1.66\ 10^{-8}$ |
| xx | $4.91\ 10^{-8}$ |

In the above Table for $IC_{50}$ the concentration of a compound is intended which reduces specific binding of $^3$H-nitrendipine to 50% of its maximal value.

The activity on the urodynamic parameters was detected by cystometric recordings, carried out in conscious rats, according to the method of Pietra et al. (IRCS Medicinal Science, in press, 1986), similar to that described by Sjogren (Acta Pharmacol. Toxicol., 39, 177, 1976). The intravescical pressure was recorded during a continuous infusion of saline (37° C) into the urinary bladder at the constant rate of (0.15 ml/min. The bladder volume capacity (BVC), and micturition pressure (MP), were recorded before and after administration of the compounds per oral route; the perfusion started 60' after the compound administration.

As example, some of the results obtained are reported in Table 3.

Table 3

Cystometric recordings (rat)

| active compound | dose (mg/kg p.o.) | % change BVC | % change MP |
|---|---|---|---|
| i | 10 | +18 | -14 |
| | 30 | +21 | -19 |
| iii | 30 | +15 | -1 |
| v | 10 | +19 | -16 |
| | 30 | +22 | -23 |
| vi | 30 | +31 | -1 |

In the above Table BVC is the bladder volume capacity, and MP is the micturition pressure.
The following Examples illustrate the invention.

EXAMPLE 1

Preparation of 3-methyl-8-formylflavone (II).

A mixture of 59.74 g of 3-methyl-8-chlorocarbonylflavone (obtained from 3-methylflavon-8-carboxilic acid and thionyl chloride, white crystals, m.p. 156-156.5 ° C), 200 ml of xylene, 6 g of 5% palladium on barium sulfate and 0.4 ml of quinoline-S maintained under stirring and under hydrogen flow, was heated for 6-7 hours at 85-90 ° C. At the end of the reaction the mixture was cooled, diluted with chloroform (20 ml), filtered, the solvent was evaporated under vacuum and to the residue 260 ml of 20% sodium hydrogensulfite (W/V) and 600 ml of water were added. The mixture thus obtained was heated at reflux for 2 hours and then filtered while hot. The cooled filtrate was extracted several times with ethyl ether (4 x 200 ml), added of concentrated hydrogen chloride and heated to 95-100 ° C for 2 hours. After cooling, the solid thus formed was filtered and washed with water. The product (II) was obtained with a global yield equal to 78% of theoretical values (41.46 g) and can be used exactly as such for further syntheses. After recrystallization from methylterbutylether it melted at 124-126 ° C.

Procedural route a)

Dimethyl 2,6-dimethyl-4-(3-methylflavon-8-yl)-1,4-dihydropyridine-3,5-dicarboxylate (i).

A mixture of 7.94 g of 3-methyl-8-formylflavone (obtained as described above), 4.1 g of methyl acetacetate, 4.14 g of methyl 3-aminocrotonate and 22.5 ml of ethanol was heated at reflux in the darkness and under stirring for 4 hours. After cooling at room temperature, the mixture was kept overnight at 5 ° C, then the insoluble thus obtained was filtered, washed with ice-cooled ethanol (3 x 5 ml) and crystallized first from ethanol and then from methanol until constant m.p. (245-249 ° C). By concentration of crystallization liquors, further 5.33 g of product were obtained which, combined to the 4.05 g previously obtained, raised the global yield of the title compound to 9.38 g.

Bis 2,N-piperidinoethyl 2,6-dimethyl-4-(3-methylflavon-8-yl)-1,4-dihydropyridine-3,5-dicarboxylate (ii). melting at 210-216 ° C after crystallization from acetonitrile/ethyl acetate, was prepared operating as described above, but employing instead of methyl acetacetate and methyl 3-aminocrotonate, 2,N-piperidinoethyl acetacetate and 2,N-piperidinoethyl 3-aminocrotonate, prepared following used procedures.

EXAMPLE 2

Procedural route b)

Step 1

Preparation of beta-chloroethyl 2-(3-methylflavon-8-methyliden)acetacetate.

A mixture of 52.8 g of (II), 32,84 g of betachloroethylacetacetate and 400 ml of toluene was saturated at 0°/+5°C with gaseous hydrochloric acid. The mixture was let to stand at room temperature for 24-48 hours, then nitrogen was bubbled to drive the acid away and the solvent was evaporated. The residue was washed first with hot ethyl ether (2 x 100 ml) and then with cold ethyl ether (1 x 100 ml) to obtain 70 g of the title compound as sole stereoisomer melting at 130-131°C.

Step 2

Isopropyl beta-chloroethyl 2,6-dimethyl-4-(3-methylflavon-8-yl)-1,4-dihydropyridine-3,5-dicarboxylate (iii).

A mixture of 51.35 g of the above described acetacetate and 7.15 ml of isopropyl 3-aminocrotonate in 94 ml of isopropanol, maintained under stirring and sheltered from the light, was heated to 80°C for 2 hours. After cooling, the solid precipitate thus formed was separated by filtration and washed with isopropanol (3 x 30 ml) to obtain 53 g of the title compound that was used as such for further reactions. A sample crystallized from acetone for the elemental analysis, melted at 235-237°C.

Step 3

Isopropyl 2,N-piperidinoethyl 2,6-dimethyl-4-(3-methylflavon-8-yl)-1,4-dihydropyridine-3,5-dicarboxylate (iv).

A mixture of the above described compound and 2.13 g of piperidine in 20 ml of anhydrous dimethylformamide, maintained under stirring, in nitrogen atmosphere and in the darkness, was heated at 100°C for 3 hours, then cooled and poured in 100 ml of ice-cold water. The solid precipitate thus formed was filtered, washed with water and, after drying, chromatographed on a silica-gel column (ethyl acetate-methanol 9:1 as eluent). After evaporation of the solvent, the title compound was obtained as residue. Yield 2.92 g after crystallization from ethyl acetate, m.p. 205-206°C.

Operating as described at step 3 of this Example, but employing N-methylbenzylamine instead of piperidine, isopropyl-2-(N-benzyl-N-methylamino)ethyl 2,6-dimethyl-4-(3-methylflavon-8-yl)-1,4-dihydropyridine-3,5-dicarboxylate (v) was obtained, melting at 170-172°C (ethanol).

In the same manner, but employing N-methyl 3,3-diphenylpropylamine instead of piperidine, isopropyl 2-(N-(3,3-diphenylpropyl)-N-methylamino)ethyl 2,6-dimethyl-4-(3-methylflavon-8-yl)-1,4-dihydropyridine-3,5-dicarboxylate (vi) was obtained, melting at 151-153°C (ethanol).

EXAMPLE 3

Procedural route c)

Step 1

Preparation of methyl 2-(3-methylflavon-8-methyliden)acetacetate.

Using the procedure described in the step 1 of the Example 2 for the preparation of beta-chloroethyl 2-(3-methylflavon-8-methyliden)acetacetate, but starting from a solution comprising 39.6 g of (II) and 17.4 g of methylacetacetate in 150 ml of chloroform, the title compound (38.55 g) was obtained.

Step 2

Methyl 2-cyanoethyl 2,6-dimethyl-4-(3-methylflavon-8-yl)-1,4-dihydropyridine-3,5-dicarboxylate (vii).

Operating as described in the step 2 of the Example 2, but using a mixture of 36.2 g of the above acetacetate and 15.42 g of 2-cyanoethyl 3-aminocrotonate in 60 ml of isopropanol, 42 g of the title compound were obtained that was employed for the subsequent reaction without further purifications.
A sample, crystallized from methylenchloride/ethyl ether for the elemental analysis, melted at 190-192°C.

Step 3

Preparation of 2,6-dimethyl-4-(3-methylflavon-8-yl)-1,4-dihydropyridine-3,5-dicarboxyllc acid methylester hydrate

To a solution of 25 g of the 2-cyanoethyl derivative described above, in 250 ml of dimethoxyethane maintained at 20-25°C under stirring and in the darkness, first 150 ml of water and then 100 ml of N sodium hydroxide were added dropwise. The mixture was maintained at the same temperature for about 2 hours, then it was extracted with chloroform (2 x 6.5 ml) and then with ethyl ether (1 x 6.5 ml). The aqueous phase was added with ice and acidified to pH 2-3 with hydrochloric acid, and the precipitate thus formed was filtered, washed, dried and crystallized from methanol to give 11.67 g of the title compound melting at 159-160°C.

Step 4

Methyl ethyl 2,6-dimethyl-4-(3-methylflavon-8-yl)-1,4-dihydropyridine-3,5-dicarboxylate (viii).

A mixture of 9.26 g of the acid prepared as described above, 4.6 g of dicyclohexylcarbodiimide, 0.24 g of 4-N,N-dimethylaminopyridine and 9.2 ml of ethanol in 40 ml of anhydrous dimethylformamide, maintained under stirring in the darkness and in nitrogen atmosphere, was heated at 80°C for 24 hours. At the end of the reaction, after cooling, the mixture was filtered and the filtrate was poured in 200 ml of ice-cold water. The precipitate thus formed was collected on filter, washed with water, dried and crystallized from ethanol, to give 6.4 g of the title compound melting at 226-228°C.

Operating as described in the above step 4, but starting from 0.92 g of the acid described in the step 3, and employing 0.3 g of bromoethanol and 0.276 g of anhydrous potassium carbonate instead of dicyclohexylcarbodiimide and of the N,N-dimethylaminopyridine, 0.33 g of the methyl 2-hydroxyethyl 2,6-dimethyl-4-(3-methylflavon-8-yl)-1,4-dihydropyridine-3,5-dicarboxylate (ix) were obtained, melting at 201-203°C.

In the same manner, but employing a suitable halogen-derivative instead of bromoethanol, the following compounds were prepared:

Methyl isopropyl 2,6-dimethyl-4-(3-methylflavon-8-yl)-1,4-dihydropyridine-3,5-dicarboxylate (x) (m.p. 209-211°C).

Methyl n-butyl 2,6-dimethyl-4-(3-methylflavon-8-yl)-1,4-dihydropyridine-3,5-dicarboxylate (xi) (m.p. 196-198°C).

Methyl propargyl 2,6-dimethyl-4-(3-methylflavon-8-yl)-1,4-dihydropyridine-3,5-dicarboxylate (xii) (m.p. 213-216°C).

Methyl allyl 2,6-dimethyl-4-(3-methylflavon-8-yl)-1,4-dihydropyridine-3,5-dicarboxylate (xiii) (m.p. 219-221°C).

Methyl alpha-methylallyl 2,6-dimethyl-4-(3-methylflavon-8-yl)-1,4-dihydropyridine-3,5-dicarboxylate (xiv) -(m.p. 187-192°C).

Methyl benzyl 2,6-dimethyl-4-(3-methylflavon-8-yl)-1,4-dihydropyridine-3,5-dicarboxylate (xv) (m.p. 204-207°C).

Methyl 2-oxopropyl 2,6-dimethyl-4-(3-methylflavon-8-yl) -1,4-dihydropyridine-3,5-dicarboxylate (xvi) (m.p. 182-183°C).

Methyl 2,3-dihydroxypropyl 2,6-dimethyl-4-(3-methylflavon-8-yl)-1,4-dihydropyridine-3,5-dicarboxylate (xvii) -(m.p. 168-170°C).

Methyl 2-ethoxyethyl 2,6-dimethyl-4-(3-methylflavon-8-yl)-1,4-dihydropyridine-3,5-dicarboxylate (xviii) (m.p. 188-191°C).

Methyl 2-ethylthioethyl 2,6-dimethyl-4-(3-methylflavon-8-yl)-1,4-dihydropyridine-3,5-dicarboxylate (xix) (m.p. 165-167°C).

Methyl 2-acetoxyethyl 2,6-dimethyl-4-(3-methylflavon-8-yl)-1,4-dihydropyridine-3,5-dicarboxylate (xx) (m.p. 183-186°C).

Methyl 2-phenylethyl 2,6-dimethyl-4-(3-methylflavon-8-yl)-1,4-dihydropyridine-3,5-dicarboxylate (xxi) (m.p. 196-199°C).

Methyl 2-(N,N-dimethylamino)ethyl 2,6-dimethyl-4-(3-methylflavon-8-yl)-1,4-dihydropyridine-3,5-dicarboxylate (xxii ) (m.p. 208-210°C).

EXAMPLE 4

Methyl 2-aminoethyl 2,6-dimethyl-4-(3-methylflavon-8-yl)-1,4-dihydropyridine-3,5-dicarboxylate (xxiii).

To a suspension of 5,34 g of the acid prepared as described in the step 3 of the Example 3, in 24 ml of anhydrous methylene chloride and 6 ml of anhydrous dimethylformamide maintained under stirring in nitrogen atmosphere at -5/0 ° C, in 5 minutes and dropwise 1.64 g of thionyl chloride were added and after 30 minutes at 0/5 ° C 1.35 g of 2-aminoethanol hydrochloride. After standing 3 h at 20-25 ° C, the mixture was cooled again on an ice-cold bath and 50 ml of 10% sodium carbonate were added dropwise. The organic phase was separated and washed with water (1 x 30 ml) with 25% acetic acid (V/V) and then with (2 x 60 ml) of water. The acidic and aqueous layers were combined, washed with ethyl ether (2 x 60 ml) and alkalinized with concentrated sodium hydroxide. After standing, the precipitate was collected, washed with water, dried and purified by silica gel chromatography, eluting with a mixture of ethyl acetate/methanol/methanolic ammonia (about 7.5 N) in ratio of 92:8:4.

3.81 g of the title compound melting at 188-192 ° C, were obtained after crystallization from methylene chloride/ethyl ether.

Operating as described above, but employing a suitable alcohol instead of 2-aminoethanol, the following compounds were obtained:

Methyl 2-(N-methyl-N-phenylamino)ethyl 2,6-dimethyl-4-(3-methylflavon-8-yl)-1,4-dihydropyridine-3,5-dicarboxylate (xxiv) (m.p. 177-182 ° C).

Methyl 2-(N-methyl-N-cyclohexilamino)ethyl 2,6-dimethyl-4-(3-methylflavon-8-yl)-1,4-dihydropyridine-3,5-dicarboxylate (xxv) (m.p. 153-155 ° C).

Methyl 2,N-morpholinoethyl 2,6-dimethyl-4-(3-methylflavon-8-yl)-1,4-dihydropyridine-3,5-dicarboxylate (xxvi) (m.p. 215-217 ° C).

Methyl 2,N-imidazolylethyl 2,6-dimethyl-4-(3-methylflavon-8-yl)-1,4-dihydropyridine-3,5-dicarboxylate (xxvii) (m.p. 198-200 ° C).

Methyl 2,N-piperidinoethyl 2,6-dimethyl-4-(3-methylflavon-8-yl)-1,4-dihydropyridine-3,5-dicarboxylate (xxviii) (m.p. 189-193 ° C).

Methyl 2,N-piperidino-1,1-dimethylethyl 2,6-dimethyl-4-(3-methylflavon-8-yl)-1,4-dihydropyridine-3,5-dicarboxylate (xxix) (m.p. 212-214 ° C).

Methyl 2,N-methylaminoethyl 2,6-dimethyl-4-(3-methylflavon-8-yl)-1,4-dihydropyridine-3,5-dicarboxylate (xxx) (m.p. 180-182 ° C).

Methyl 2,N-(N'-methyl)piperazinoethyl 2,6-dimethyl-4-(3-methylflavon-8-yl)-1,4-dihydropyridine-3,5-dicarboxylate (xxxi) (m.p. 181-185 ° C).

Methyl terbutyl 2,6-dimethyl-4-(3-methylflavon-8-yl)-1,4-dihydropyridine-3,5-dicarboxylate (xxxii) (m.p. 222-225 ° C).

Methyl cyclohexyl 2,6-dimethyl-4-(3-methylflavon-8-yl)-1,4-dihydropyridine-3,5-dicarboxylate (xxxiii) (m.p. 137-139 ° C).

Methyl phenyl 2,6-dimethyl-4-(3-methylflavon-8-yl)-1,4-dihydropyridine-3,5-dicarboxylate (xxxiv) (m.p. 211-213 ° C).

Methyl 2-ethylsulfonylethyl 2,6-dimethyl-4-(3-methylflavon-8-yl)-1,4-dihydropyridine-3,5-dicarboxylate (xxxv) (m.p. 195-200 ° C).

EXAMPLE 5

Step 1

Preparation of methyl 4,4-dimethoxy-2-(3-methylflavon-8-methyliden)acetacetate.

A mixture comprising 2.64 g of (II), 2.1 g of methyl 4,4-dimethoxyacetacetate, 0.034 ml of acetic acid, 0.038 ml of piperidine and 8 ml of benzene, was heated refluxed under stirring for 12 h, while the azeotropic mixture was distilled off. After cooling, the solution was washed first with 5% sodium bicarbonate, then with water and dried over anhydrous sodium sulfate. After evaporation of the solvent, the residue was purified by silica gel chromatography eluting with petroleum ether/ethyl acetate first in 4:1 ratio and then in 3:2 ratio. The fractions containing the pure product were collected and the solvents were evaporated to give 2 g of the title product that was employed for the subsequent reaction without further purification.

A sample crystallized from ethyl acetate adding petroleum ether, melted at 124-126 ° C.

Step 2

Dimethyl 2-dimethoxymethyl-6-methyl-4-(3-methylflavon-8-yl)-1,4-dihydropyridine-3,5-dicarboxylate (xxxvi).

A mixture of 1.1 g of the acetacetate described in the above step, 0.34 g of methyl 3-aminocrotonate and 1.5 ml of N,N-dimethylformamide, maintained under nitrogen atmosphere in the darkness, was heated at 80 ° C under stirring for 5 h and then at 100-105 ° C for further 7 h. After cooling, the mixture was diluted with about 15 ml of water and extracted with ethyl ether. The organic phase was washed with water, dried over sodium sulfate and after evaporation of the solvent, the residue was purified by silica-gel chromatography eluting with ethyl ether. The fractions containing the pure product were collected and, after evaporation of the solvent, the residue was crystallized from ethyl acetate adding petroleum ether, to give 0.32 g of the title product melting at 166-168 ° C.

Step 3

Dimethyl 2-formyl-6-methyl-4-(3-methylflavon-8-yl)-1,4-dihydropyridine-3,5-dicarboxylate (xxxvii)

A solution of 10.15 g of the dimethoxymethyl derivative, obtained as described in the above step 2, in 88 ml of acetone and 10 ml of 6 N hydrochloric acid, was stirred under nitrogen atmosphere and in the darkness for 5 h at 18-22 ° C. After dilution with 120 ml of water, 5.04 g of sodium bicarbonate were added portionwise and then the most of the acetone was evaporated off, and the solid collected by filtration, was purified by silica-gel chromatography, eluting with methylene chloride/ethyl ether 9:1. The fractions containing the pure product were collected and, after evaporation of the solvent, the residue was crystallized from acetonitrile to give 4.6 g of the title product melting at 216 ° C.

EXAMPLE 6

Dimethyl 2-hydroxymethyl-6-methyl-4-(3-methylflavon-8-yl)-1,4-dihydropyridine-3,5-dicarboxylate (xxxviii).

To a mixture comprising 3.3 g of the 2-formyl derivative prepared as described in the step 3 of the previous Example, 52 ml of ethanol, 18 ml of methylene chloride, maintained under stirring at 0 ° C, in nitrogen atmosphere and in the darkness, 0.28 g of sodium borohydryde were added portionwise. The solution was kept in these conditions for 2 h, then it was acidified with 50% acetic acid, most of the solvents was evaporated under vacuum and to the residue first 70 ml of water and then 5% of sodium bicarbonate were added, until the reaction was alkaline. The solid thus formed was collected by filtration and crystallized from acetonitrile to give 1.7 g of the title product melting at 231-233 ° C.

EXAMPLE 7

Dimethyl 2-cyano-6-methyl-4-(3-methylflavon-8-yl)-1,4-dihydropyridine-3,5-dicarboxylate (xxxix).

A mixture comprising 0.95 g of the 2-formyl derivative prepared as described in the previous Example, 0.17 g of hydroxylamine hydrochloride, 0.245 g of anhydrous sodium acetate and 3.7 ml of acetic acid, was maintained under stirring at 18-22 ° C in the darkness and in nitrogen atmosphere for 4 h. At the end 0.75 g of acetic anhydride were added, and the mixture was let to 18-22 ° C for 1 h, then it was heated at 95-100 ° C for 4 h. After cooling, the most of the acetic acid was evaporated under vacuum, and the residue was diluted with water, neutralized with 5% sodium bicarbonate and then extracted with ethyl acetate. The crude, obtained after evaporation of the organic phase, was purified by silica gel chromatography eluting with methylene chloride/ethyl acetate (9:1). The fractions containing the pure product were collected, the solvents were evaporated off and the residue crystallized from N,N-dimethylformamide/water to give 0.3 g of the title product melting over 250 ° C.

## Claims

**1.** A compound of general formula

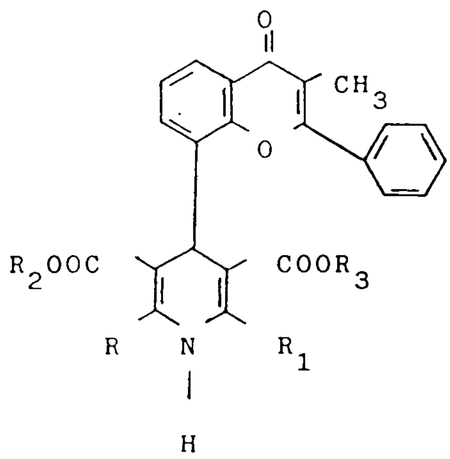

(I)

wherein R and $R_1$ can be equal or different and are alkyl containing from 1 to 4 carbon atoms, hydroxyalkyl having 1-4 carbon atoms, formylalkyl or cyanoalkyl, $R_2$ and $R_3$ could be equal or different and are straight or branched alkyl containing from 1 to 6 carbon atoms, straight or branched alkenyl containing from 2 to 6 carbon atoms, alkynyl containing from 2 to 6 carbon atoms, cycloalkyl having 5-7 carbon atoms, aralkyl, phenyl, cyanoalkyl, haloalkyl, mono- or poly-hydroxyalkyl, alkoxyalkyl, alkylthioalkyl, alkylsulfonylalkyl, acyloxyalkyl, acylalkyl, $R_4R_5$N-alkyl straight or branched having 2-6 carbon atoms, where $R_4$ and $R_5$ are equal or different and are hydrogen, alkyl, cycloalkyl, aralkyl, phenyl, 3,3-diphenylpropyl, their optical isomers and diastereoisomers, and pharmaceutically acceptable salts thereof.

**2.** Compound of formula (I) according to claim 1 selected among:
dimethyl 2,6-dimethyl-4-(3-methylflavon-8-yl)-1,4-dihydropyridine-3,5-dicarboxylate,
bis 2,N-piperidinoethyl 2,6-dimethyl-4-(3-methylflavon-8-yl)-1,4-dihydropyridine-3,5-dicarboxylate,
dimethyl 2-formyl-6-methyl-4-(3-methylflavon-8-yl)-1,4-dihydropyridine-3,5-dicarboxylate,
dimethyl 2-hydroxymethyl-6-methyl-4-(3-methylflavon-8-yl)-1,4-dihydropyridine-3,5-dicarboxylate,
dimethyl 2-cyano-6-methyl-4-(3-methylflavon-8-yl)-1,4-dihydropyridine-3,5-dicarboxylate.

**3.** Compound of formula (I) according to claim 1 selected among:
isopropyl beta-chloroethyl 2,6-dimethyl-4-(3-methylflavon-8-yl)-1,4-dihydropiridine-3,5-dicarboxylate,
isopropyl 2,N-piperidinoethyl 2,6-dimethyl-4-(3-methylflavon-8-yl)-1,4-dihydropyridine-3,5-dicarboxylate,
isopropyl 2-(N-benzyl-N-methylamino)ethyl 2,6-dimethyl-4-(3-methylflavon-8-yl)-1,4-dihydropyridine-3,5-dicarboxylate,
isopropyl 2-(N-(3,3-diphenylpropyl)-N-methylamino)ethyl 2,6-dimethyl-4-(3-methylflavon-8-yl)-1,4-dihydropyridine-3,5-dicarboxylate,
methyl 2-cyanoethyl 2,6-dimethyl-4-(3-methylflavon-8-yl)-1,4-dihydropyridine-3,5-dicarboxylate,
methyl ethyl 2,6-dimethyl-4-(3-methylflavon-8-yl)-1,4-dihydropyridine-3,5-dicarboxylate,
methyl 2-hydroxyethyl 2,6-dimethyl-4-(3-methylflavon-8-yl)-1,4-dihydropyridine-3,5-dicarboxylate,
methyl isopropyl 2,6-dimethyl-4-(3-methylflavon-8-yl)-1,4-dihydropyridine-3,5-dicarboxylate,
methyl n-butyl 2,6-dimethyl-4-(3-methylflavon-8-yl)-1,4-dihydropyridine-3,5-dicarboxylate,
methyl propargyl 2,6-dimethyl-4-(3-methylflavon-8-yl)-1,4-dihydropyridine-3,5-dicarboxylate,
methyl allyl 2,6-dimethyl-4-(3-methylflavon-8-yl)-1,4-dihydropyridine-3,5-dicarboxylate,
methyl alpha-methylallyl 2,6-dimethyl-4-(3-methylflavon-8-yl)-1,4-dihydropyridine-3,5-dicarboxylate,
methyl benzyl 2,6-dimethyl-4-(3-methylflavon-8-yl)-1,4-dihydropyridine-3,5-dicarboxylate,
methyl 2-oxopropyl 2,6-dimethyl-4-(3-methylflavon-8-yl) -1,4-dihydropyridine-3,5-dicarboxylate,
methyl 2,3-dihydroxypropyl 2,6-dimethyl-4-(3-methylflavon-8-yl)-1,4-dihydropyridine-3,5-dicarboxylate,
methyl 2-ethoxyethyl 2,6-dimethyl-4-(3-methylflavon-8-yl)-1,4-dihydropyridine-3,5-dicarboxylate,
methyl 2-ethylthioethyl 2,6-dimethyl-4-(3-methylflavon-8-yl)-1,4-dihydropyridine-3,5-dicarboxylate,
methyl 2-acetoxyethyl 2,6-dimethyl-4-(3-methylflavon-8-yl)-1,4-dihydropyridine-3,5-dicarboxylate,
methyl 2-phenylethyl 2,6-dimethyl-4-(3-methylflavon-8-yl)-1,4-dihydropyridine-3,5-dicarboxylate,
methyl 2-(N,N-dimethylamino)ethyl 2,6-dimethyl-4-(3-methylflavon-8-yl)-1,4-dihydropyridine-3,5-dicarboxylate,
methyl 2-aminoethyl 2,6-dimethyl-4-(3-methylflavon-8-yl)-1,4-dihydropyridine-3,5-dicarboxylate,
methyl 2-(N-methyl-N-phenylamino)ethyl 2,6-dimethyl-4-(3-methylflavon-8-yl)-1,4-dihydropyridine-3,5-dicarboxylate,
methyl 2-(N-methyl-N-cyclohexilamino)ethyl 2,6-dimethyl-4-(3-methylflavon-8-yl)-1,4-dihydropyridine-3,5-dicarboxylate,
methyl 2,N-morpholinoethyl 2,6-dimethyl-4-(3-methylflavon-8-yl)-1,4-dihydropyridine-3,5-dicarboxylate,
methyl 2,N-imidazolylethyl 2,6-dimethyl-4-(3-methylflavon-8-yl)-1,4-dihydropyridine-3,5-dicarboxylate,
methyl 2,N-piperidinoethyl 2,6-dimethyl-4-(3-methylflavon-8-yl)-1,4-dihydropyridine-3,5-dicarboxylate,
methyl 2,N-piperidino-1,1-dimethylethyl 2,6-dimethyl-4-(3-methylflavon-8-yl)-1,4-dihydropyridine-3,5-dicarboxylate,
methyl 2,N-methylaminoethyl 2,6-dimethyl-4-(3-methylflavon-8-yl)-1,4-dihydropyridine-3,5-dicarboxylate,
methyl 2,N-(N'-methyl)piperazinoethyl 2,6-dimethyl-4-(3-methylflavon-8-yl)-1,4-dihydropyridine-3,5-dicarboxylate,
methyl terbutyl 2,6-dimethyl-4-(3-methylflavon-8-yl)-1,4-dihydropyridine-3,5-dicarboxylate,
methyl cyclohexyl 2,6-dimethyl-4-(3-methylflavon-8-yl)-1,4-dihydropyridine-3,5-dicarboxylate,
methyl phenyl 2,6-dimethyl-4-(3-methylflavon-8-yl)-1,4-dihydropyridine-3,5-dicarboxylate,
methyl 2-ethylsulfonylethyl 2,6-dimethyl-4-(3-methylflavon-8-yl)-1,4-dihydropyridine-3,5-dicarboxylate.

**4.** Process for preparing a compound of formula (I) according to claim 1, characterized in that
a) an aldheyde of formula

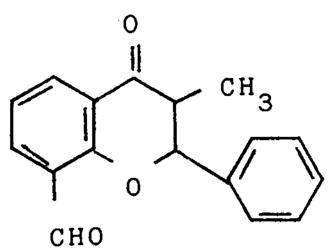

(II)

(hereinafter identified as (II) ) is reacted with a ketoester of formula

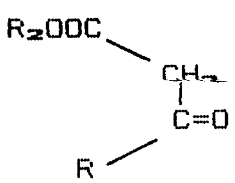

(III)

and with an enamine of formula

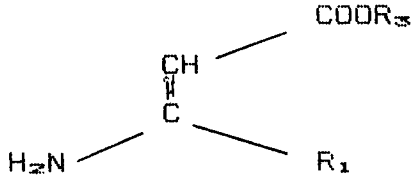

(IV)

wherein R, $R_1$, $R_2$, and $R_3$ have the above mentioned meanings and R, $R_1$ can be transformed by means of known procedures in formylalkyl, hydroxyalkyl, or cyanoalkyl groups, or alternatively:
b) (II) is treated with a compound of formula

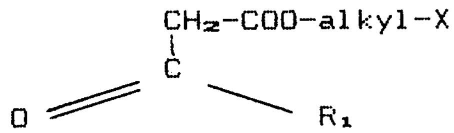

(V)

wherein for $R_1$ is worth that said in a) and X is a halogen atom, and the arylidenederivative thus obtained is reacted with an enamine of formula

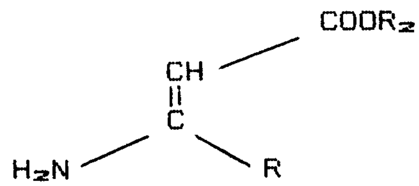

(VI)

wherein R, and $R_2$ have the above mentioned meanings, and at last optionally an amine of formula $R_4R_5NH$ is added, wherein $R_4$ and $R_5$ are above defined or alternatively

c) the aldehyde (II) is reacted with a ketoester of formula (III) and then, to the obtained arylidenederivative, an enamine of formula

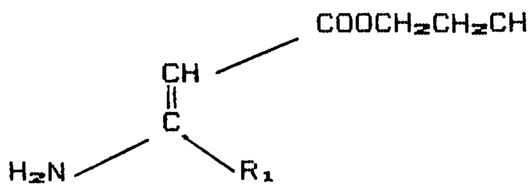

(VII)

is added, wherein R, $R_1$, and $R_2$ have the above meanings or is worth what said in a), and the cyanoethylester thus obtained is hydrolyzed to give the acid of formula

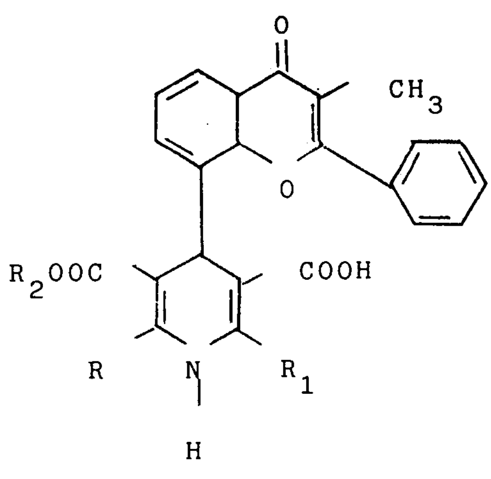

(VIII)

that is esterified following known procedures with a derivative of formula $R_3OH$ or $R_3X$, where X is a halogen atom and $R_3$ has the above described meaning.

**5.** Process according to claim 4, characterized in that the route a) is performed in a lower alcohol, preferably ethanol for 2-5 hours at the reflux temperature of the employed alcohol.

**6.** Process according to claim 4, characterized in that the first step of the route b) is performed in a chlorinated hydrocarbon, preferably chloroform, the following addition of (VI) in a lower alcohol, preferably isopropanol, and the optional reaction with the amine $R_4R_5NH$ in dimethylformamide, at high temperature, preferably about at 100 ° C.

**7.** Process according to claim 4, characterized in that the first step of the route c) is performed in a chlorinated hydrocarbon, preferably chloroform, then the addition of the enamine in alcohol, preferably isopropanol, the hydrolysis of the acid in dimethoxyethane and the esterification in dimethylformamide.

**8.** Process according to claims 4-7 characterized in that the transformation of R or $R_1$ in formylalkyl group is performed by cyclization of a compound of formula (VII) or (III) in which R or $R_1$ is geminal dialkoxyalkyl and then by further hydrolysis, with hydrochloric acid in acetone, of the obtained 1,4-dihydropyridinic ring, having in R or $R_1$ the above mentioned dialkoxyalkyl group.

**9.** Process according to claims 4-8 characterized in that the transformation of R or $R_1$ in hydroxyalkyl group is performed by reduction of the corresponding formylalkyl group with sodium borohydryde in alcohol, preferably ethanol.

**10.** Process according to claims 4-8 characterized in that the transformation of R or $R_1$ in cyanoalkyl group is performed by reaction of the corresponding formylalkyl group with hydroxylamine and acetic anhydride.

**11.** Pharmaceutical compositions with a calcium-antagonistic activity and/or an activity relaxing the smooth musculature (in particular the bladder), comprising a compound of formula (I) according to claim 1 and a suitable support and diluent.

**12.** Pharmaceutical compositions according to claim 11 for the oral or parenteral administration, to be employed at the usual doses for such kind of drugs.

## Revendications

**1.** Composé de formule générale:

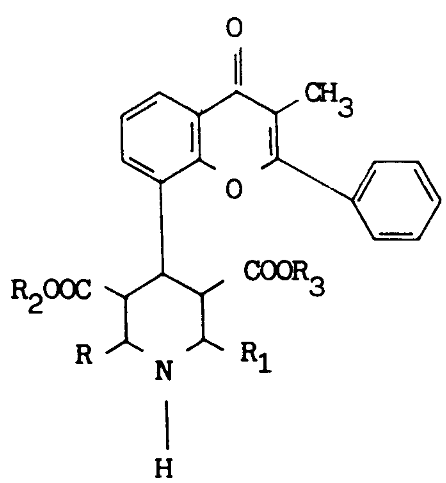

(I)

dans laquelle R et $R_1$ peuvent être égaux ou différents et sont un alkyle contenant 1 à 4 atomes de carbone, un hydroxyalkyle contenant 1 à 4 atomes de carbone, un formylalkyle ou cyanoalkyle, $R_2$ et $R_3$ peuvent être égaux ou différents et sont un alkyle linéaire ou ramifié contenant 1 à 6 atomes de carbone, un alkényle linéaire ou ramifié contenant 2 à 6 atomes de carbone, un alkynyle contenant 2 à 6 atomes de carbone, un cycloalkyle ayant 5 à 7 atomes de carbone, un aralkyle, phényle, cyanoalkyle, haloalkyle, mono- ou polyhydroxyalkyle, alkoxyalkyle, alkylthioalkyle, alkylsulfonylalkyle, acyloxyalkyle, acylalkyle, un $R_4R_5N$-alkyle linéaire ou ramifié ayant 2 à 6 atomes de carbone, où $R_4$ et $R_5$ sont égaux ou différents et sont hydrogène, alkyle, cycloalkyle, aralkyle, phényle, 3,3-diphénylpropyle, leurs isomères optiques et diastéréoisomères, et leurs sels pharmaceutiquement acceptables.

**2.** Composé de formule (I) selon la revendication 1, lequel est choisi parmi:
diméthyle 2,6-diméthyle-4-(3-méthylflavone-8-yl)-1,4-dihydropyridine-3,5-dicarboxylate,
bis 2,N-pipéridinoéthyle 2,6-diméthyle-4-(3-méthylflavone-8-yl)-1,4-dihydropyridine-3,5-dicarboxylate,

diméthyle 2-formyle-6-méthyle-4-(3-méthylflavone-8-yl)-1,4-dihydropyridine-3,5-dicarboxylate,
diméthyle-2-hydroxyméthyle-6-méthyle-4-(3-méthylflavone-8-yl)-1,4-dihydropyridine-3,5-dicarboxylate,
diméthyle 2-cyano-6-méthyle-4-(3-méthylflavone-8-yl)-1,4-dihydropyridine-3,5-dicarboxylate.

**3.** Composé de formule (I) selon la revendication 1, choisi parmi:
isopropyle bêta-chloroéthyle 2,6-diméthyle-4-(3-méthylflavone-8-yl)-1,4-dihydropyridine-3,5-dicarboxylate,
isopropyle 2,N-pipéridinoéthyle 2,6-diméthyle-4-(3-méthylflavone-8-yl)-1,4-dihydropyridine-3,5-dicarboxylate,
isopropyle 2-(N-benzyle-N-méthylamino)éthyle 2,6-diméthyle-4-(3-méthylflavone-8-yl)-1,4-dihydropyridine-3,5-dicarboxylate,
isopropyle 2-(N-(3,3-diphénylpropyle)-N-méthylamino) éthyle 2,6-diméthyle-4-(3-méthylflavone-8-yl)-1,4-dihydropyridine-3,5-dicarboxylate,
méthyle 2-cyanoéthyle 2,6-diméthyle-4-(3-méthylflavone-8-yl)-1,4-dihydropyridine-3,5-dicarboxylate,
méthyle éthyle 2,6-diméthyle-4-(3-méthylflavone-8-yl)-1,4-dihydropyridine-3,5-dicarboxylate,
méthyle 2-hydroxyéthyle 2,6-diméthyle-4-(3-méthylflavone-8-yl)-1,4-dihydropyridine-3,5-dicarboxylate,
méthyle isopropyle 2,6-diméthyle-4-(3-méthylflavone-8-yl)-1,4-dihydropyridine-3,5-dicarboxylate,
méthyle n-butyle 2,6-diméthyle-4-(3-méthylflavone-8-yl)-1,4-dihydropyridine-3,5-dicarboxylate,
méthyle propargyle 2,6-diméthyle-4-(3-méthylflavone-8-yl)-1,4-dihydropyridine-3,5-dicarboxylate,
méthyle allyle 2,6-diméthyle-4-(3-méthylflavone-8-yl)-1,4-dihydropyridine-3,5-dicarboxylate,
méthyle alpha-méthylallyle 2,6-diméthyle-4-(3-méthylflavone-8-yl)-1,4-dihydropyridine-3,5-dicarboxylate,
métyle benzyle 2,6-diméthyle-4-(3-méthylflavone-8-yl)-1,4-dihydropyridine-3,5-dicarboxylate,
méthyle 2-oxopropyle 2,6-diméthyle-4-(3-méthylflavone-8-yl)-1,4-dihydropyridine-3,5-dicarboxylate,
méthyle 2,3-dihydroxypropyle 2,6-diméthyle-4-(3-méthylflavone-8-yl)-1,4-dihydropyridine-3,5-dicarboxylate,
méthyle 2-éthoxyéthyle 2,6-diméthyle-4-(3-méthylflavone-8-yl)-1,4-dihydropyridine-3,5-dicarboxylate,
méthyle 2-éthylthioéthyle 2,6-diméthyle-4-(3-méthylflavone-8-yl)-1,4-dihydropyridine-3,5-dicarboxylate,
méthyle 2-acétoxyéthyle 2,6-diméthyle-4-(3-méthylflavone-8-yl)-1,4-dihydropyridine-3,5-dicarboxylate,
méthyle 2-phényléthyle 2,6-diméthyle-4-(3-méthylflavone-8-yl)-1,4-dihydropyridine-3,5-dicarboxylate,
méthyle 2-(N,N-diméthylamino)éthyle 2,6-diméthyle-4-(3-méthylflavone-8-yl)-1,4-dihydropyridine-3,5-dicarboxylate,
méthyle 2-aminoéthyle 2,6-diméthyle-4-(3-méthylflavone-8-yl)-1,4-dihydropyridine-3,5-dicarboxylate,
méthyle 2-(N-méthyle-N-phénylamino)éthyle 2,6-diméthyle-4-(3-méthylflavone-8-yl)-1,4-dihydropyridine-3,5-dicarboxylate,
méthyle 2-(N-méthyle-N-cyclohexylamino)éthyle 2,6-diméthyle-4-(3-méthylflavone-8-yl)-1,4-dihydropyridine-3,5-dicarboxylate,
méthyle 2,N-morpholinoéthyle 2,6-diméthyle-4-(3-méthylflavone-8-yl)-1,4-dihydropyridine-3,5-dicarboxylate,
méthyle 2,N-imidazolyléthyle 2,6-diméthyle-4-(3-méthylflavone-8-yl)-1,4-dihydropyridine-3,5-dicarboxylate,
méthyle 2,N-pipéridinoéthyle 2,6-diméthyle-4-(3-méthylflavone-8-yl)-1,4-dihydropyridine-3,5-dicarboxylate,
méthyle 2,N-pipéridino-1,1-diméthyléthyle 2,6-diméthyle-4-(3-méthylflavone-8-yl)-1,4-dihydropyridine-3,5-dicarboxylate,
méthyle 2,N-méthylaminoéthyle 2,6-diméthyle-4-(3-méthylflavone-8-yl)-1,4-dihydropyridine-3,5-dicarboxylate,
méthyle 2,N-(N'-méthyle)pipérazinoéthyle 2,6-diméthyle-4-(3-méthylflavone-8-yl)-1,4-dihydropyridine-3,5-dicarboxylate,
méthyle terbutyle 2,6-diméthyle-4-(3-méthylflavone-8-yl)-1,4-dihydropyridine-3,5-dicarboxylate,
méthyle cyclohexyle 2,6-diméthyle-4-(3-méthylflavone-8-yl)-1,4-dihydropyridine-3,5-dicarboxylate,
méthyle phényle 2,6-diméthyle-4-(3-méthylflavone-8-yl)-1,4-dihydropyridine-3,5-dicarboxylate,
méthyle 2-éthylsulfonyléthyle 2,6-diméthyle-4-(3-méthylflavone-8-yl)-1,4-dihydropyridine-3,5-dicarboxylate.

**4.** Procédé pour la préparation d'un composé de formule (I) selon la revendication 1, caractérisé en ce que:

a) on fait réagir un aldhéyde de formule:

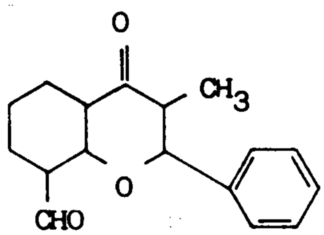

(II)

(ci-après dénommé II) avec un cétoester de formule:

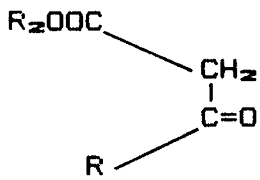

(III)

et avec une énamine de formule:

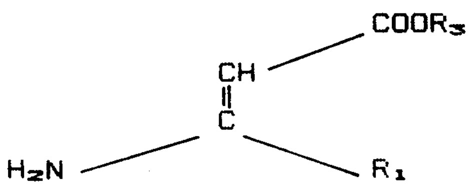

(IV)

où R, $R_1$, $R_2$, et $R_3$ ont la valeur indiquée plus haut et R, $R_1$ peuvent être transformés au moyen de procédures connues en groupes formylalkyle, hydroxyalkyle, ou cyanoalkyle, ou en alternative:
b) (II) est traité avec un composé de formule

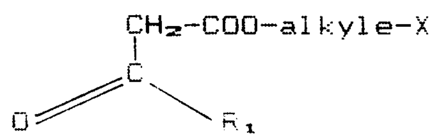

(V)

où en ce qui concerne $R_1$ est valable ce qu'on a précisé en a) et X est un atome d'halogène, et on fait réagir le dérivé d'arylidène ainsi obtenu avec une énamine de formule:

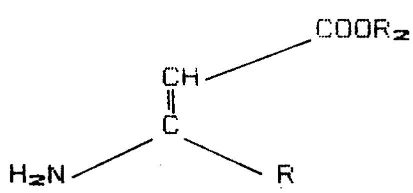

(VI)

où R et $R_2$ ont les valeurs précisées plus haut, et on y ajoute enfin facultativement une amine de formule $R_4R_5NH$, où $R_4$ et $R_5$ sont comme précédemment indiqué, ou en alternative:
c) on fait réagir l'aldhéyde (II) avec un cétoester de formule (III) et on y ajoute ensuite au dérivé d'arylidène obtenu une énamine de formule:

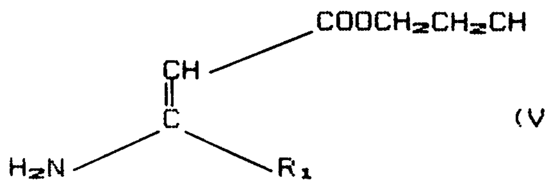

(VII)

où R, $R_1$ et $R_2$ ont les valeurs précédemment précisées ou bien est valable ce qu'on a dit au point a), et on hydrolyse ensuite le cyanoéthylester ainsi obtenu de manière à donner l'acide de formule:

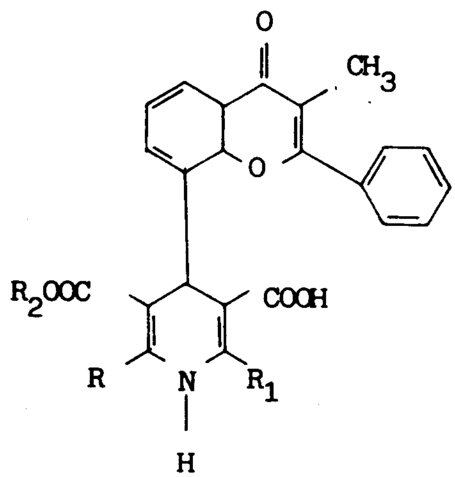

(VIII)

qui est estérifié au moyen de procédures connues, avec un dérivé de formule $R_3OH$ ou $R_3X$, ou X est un atome d'halogène et $R_3$ a la valeur précisée plus haut.

**5.** Procédé selon la revendication 4, caractérisé en ce que l'itinéraire a) est exécuté en un alcool inférieur, de préférence éthanol, pendant 2 à 5 heures, à la température de reflux de l'alcool employé.

**6.** Procédé selon la revendication 4, caractérisé en ce que la première étape de l'itinéraire b) est exécutée dans un hydrocarbure chloruré, de préférence chloroforme, alors que l'addition subséquente de (VI) a lieu dans un alcool inférieur, de préférence isopropanol et la réaction facultative avec l'amine $R_4R_5NH$ est exécutée en diméthylformamide, à une température élevée, de préférence d'environ 100 ° C.

**7.** Procédé selon la revendication 4, caractérisé en ce que la première étape de l'itinéraire c) est exécutée dans un hydrocarbure chloruré, de préférence chloroforme, puis l'addition de l'énamine a lieu en alcool, de préférence isopropanol, l'hydrolyse de l'acide est exécutée en diméthoxyéthane et l'estérification en diméthylformamide.

**8.** Procédé selon les revendications 4 à 7, caractérisé en ce que la transformation de R ou $R_1$ dans le groupe formylalkyle est exécutée par la cyclisation d'un composé de formule (VII) ou (VIII) dans lequel R ou $R_1$ est un dialkoxyalkyle double, et ensuite par l'hydrolyse, avec de l'acide chlorhydrique en acétone, de la chaîne 1,4-dihydropyridinique obtenue, qui a en R ou $R_1$ le groupe dialkoxyalkyle précité.

**9.** Procédé selon les revendications 4 à 8, caractérisé en ce que la transformation de R ou $R_1$ dans le groupe hydroxyalkyle est exécutée par la réduction du groupe formylalkyle correspondant avec du borohydrure de sodium en alcool, de préférence éthanol.

**10.** Procédé selon les revendications 4 à 8, caractérisé en ce que la transformation de R ou $R_1$ dans le groupe cyanoalkyle est exécutée faisant réagir le groupe formylalkyle correspondant avec de l'hydroxylamine et de l'anhydride acétique.

**11.** Compositions pharmaceutiques ayant une activité calcium-antagoniste et/ou une activité de relâchement sur la musculature lisse (notamment sur la vessie), comprenant un composé de formule (I) selon la revendication 1 et un support et un diluant appropriés.

**12.** Compositions pharmaceutiques selon la revendication 11 pour l'administration orale ou parentérale, à utiliser sur la base des doses habituelles pour ces types de produits pharmaceutiques.

**Patentansprüche**

**1.** Eine Verbindung der allgemeinen Formel

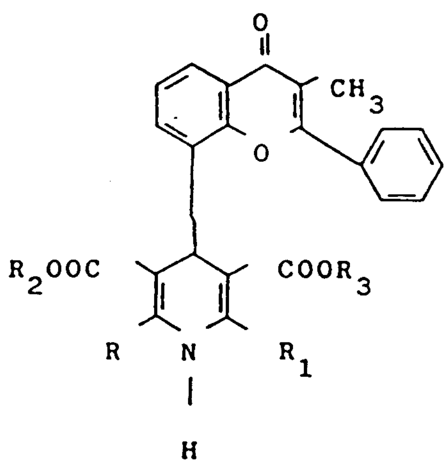

(I)

in der R und R1 gleich oder verschieden sein können und Alkyl mit 1 bis 4 Kohlenstoffatomen, Hydroxyalkyl mit 1 bis 4 Kohlenstoffatomen, Formylalkyl oder Cyanalkyl bedeuten, R2 und R3 gleich oder verschieden sein können und gerades oder verzweigtes Alkyl mit 1 bis 6 C-Atomen, gerades oder verzweigtes Alkenyl mit 2 bis 6 C-Atomen, Alkynil mit 2 bis 6 Kohlenstoffatomen, Cycloalkyl mit 5 bis 7 Kohlenstoffatomen, Aralkyl, Phenyl, Cyanalkyl, Halogenalkyl, Mono- oder Polyhydroxyalkyl, Alkoxyalkyl, Alkylthioalkyl, Alkylsulfonylalkyl, Acyloxyalkyl, Acylalkyl, R4R5 N-(gerades oder verzweigtes) C2-C6-alkyl, worin R4 und R5 gleich oder verschieden sind und Wasserstoff, Alkyl, Cycloalkyl, Aralkyl, Phenyl, 3,3-Diphenylpropyl bedeuten, ihre optische Isomeren und Diasteroisomeren sowie deren pharmazeutisch verträgliche Salze.

**2.** Verbindung del Formel (I) nach Anspruch 1 ausgewählt aus:
2,6-Dimethyl-4-(3-methylflavon-8-yl)-1,4-dihydropyridin-3,5-dicarbonsäuredimethylester,
2,6-Dimethyl-4-(3-methylflavon-8-yl)-1,4-dihydropyridin-3,5-dicarbonsäure-bis-2,N-piperidinethylester,
2-Formyl-6-methyl-4-(3-methylflavon-8-yl)-1,4-dihydropyridin-3,5-dicarbonsäuredimethylester,
2-Hydroxymethyl-6-methyl-4-(3-mehtylflavon-8-yl)-1,4-dihydropyridin-3,5-dicarbonsäuredimethylester,
2-Cyan-6-methyl-4-(3-methylflavon-8-yl)-1,4-dihydropyridin-3,5-dicarbonsäuredimethylester.

**3.** Verbindung der Formel (I) nach Anspruch 1 ausgewählt aus:
2,6-Dimethyl-4-(3-methylflavon-8-yl)-1,4-dihydropyridin-3,5-dicarbonsäureisopropyl-beta-chlorethylester,
2,6-Dimethyl-4-(3-methylflavon-8-yl)-1,4-dihydropyridin-3,5-dicarbonsäureisopropyl-2,N-piperidinethylester;
2,6-Dimethyl-4-(3-methylflavon-8-yl)-1,4-dihydropyridin-3,5-dicarbonsäureisopropyl-2-(N-benzyl-N-methylamino)-ethylester,
2,6-Dimethyl-4-(3-methylflavon-8-yl)-1,4-dihydropyridin-3,5-dicarbonsäureisopropyl-2-(N-(3,3-diphenylpropyl)-N-methylaminoethylester.
2,6-Dimethyl-4-(3-methylflavon-8-yl)-1,4-dihydropyridin-3,5-dicarbonsäuremethyl-2-cyanethylester,
2,6-Dimethyl-4-(3-methylflavon-8-yl)-1,4-dihydropyridin-3,5-dicarbonsäuremethylethylester,
2,6-Dimethyl-4-(3-methylflavon-8-yl)-1,4-dihydropyridin-3,5-dicarbonsäuremethyl-2-hydroxyethylester,
2,6-Dimethyl-4-(3-methylflavon-8-yl)-1,4-dihydropyridin-3,5-dicarbonsäuremethylisopropylester,
2,6-Dimethyl-4-(3-methylflavon-8-yl)-1,4-dihydropyridin-3,5-dicarbonsäuremethyl-n-butylester,

2,6-Dimethyl-4-(3-methylflavon-8-yl)-1,4-dihydropyridin-3,5-dicarbonsäuremethylpropargylester,
2,6-Dimethyl-4-(3-methylflavon-8-yl)-1,4-dihydropyridin-3,5-dicarbonsäuremethylallylester,
2,6-Dimethyl-4-(3-methylflavon-8-yl)-1,4-dihydropyridin-3,5-dicarbonsäuremethyl-alpha-methylallylester,
2,6-Dimethyl-4-(3-methylflavon-8-yl)-1,4-dihydropyridin-3,5-dicarbonsäuremethylbenzylester,
2,6-Dimethyl-4-(3-methylflavon-8-yl)-1,4-dihydropyridin-3,5-dicarbonsäuremethyl-2-oxopropylester,
2,6-Dimethyl-4-(3-methylflavon-8-yl)-1,4-dihydropyridin-3,5-dicarbonsäuremethyl-2,3-dihydroxypropylester,
2,6-Dimethyl-4-(3-methylflavon-8-yl)-1,4-dihydropyridin-3,5-dicarbonsäuremethyl-2-ethoxyethylester,
2,6-Dimethyl-4-(3-methylflavon-8-yl)-1,4-dihydropyridin-3,5-dicarbonsäuremethyl-2-ethylthioethyl- ester,
2,6-Dimethyl-4-(3-methylflavon-8-yl)-1,4-dihydropyridin-3,5-dicarbonsäuremethyl-2-acetoxyethylester,
2,6-Dimethyl-4-(3-methylflavon-8-yl)-1,4-dihydropyridin-3,5-dicarbonsäuremethyl-2-phenylethylester,
2,6-Dimethyl-4-(3-methylflavon-8-yl)-1,4-dihydropyridin-3,5-dicarbonsäuremethyl-2-(N,N-dimethylaminethylester,
2,6-Dimethyl-4-(3-methylflavon-8-yl)-1,4-dihydropyridin-3,5-dicarbonsäuremethyl-2-aminethylester,
2,6-Dimethyl-4-(3-methylflavon-8-yl)-1,4-dihydropyridin-3,5-dicarbonsäuremethyl-2-(N-methyl-N-phenylaminethyl-ester,
2,6-Dimethyl-4-(3-methylflavon-8-yl)-1,4-dihydropyridin-3,5-dicarbonsäuremethyl-2-(N-methyl-N-cyclohexylamino)-ethylester,
2,6-Dimethyl-4-(3-methylflavon-8-yl)-1,4-dihydropyridin-3,5-dicarbonsäuremethyl-2,N-morpholinethylester,
2,6-Dimethyl-4-(3-methylflavon-8-yl)-1,4-dihydropyridin.3.5-dicarbonsäuremethyl-2,N-imidazolylethylester
2,6-Dimethyl-4-(3-methylflavon-8-yl)-1,4-dihydropyridin-3,5-dicarbonsäuremethyl-2.N-piperidinethylester,
2,6-Dimethyl-4-(3-methylflavon-8yl)-1,4-dihydropyridin-3,5-dicarbonsäuremethyl-2,N-piperidin-1,1-dimethylethyl-ester,
2,6-Dimethyl-4-(3-methylflavon-8-yl)-1,4-dihydropyridin-3,5-dicarbonsäuremethyl-2,N-methylaminethylester,
2,6-Dimethyl-4-(3-methylflavon-8-yl)-1,4-dihydropyridin-3,5-dicarbonsäuremethyl-2,N-(N'-methyl)-piperazinethylester,
2,6-Dimethyl-4-(3-methylflavon-8-yl)-1,4-dihydropyridin-3,5-dicarbonsäuremethyl-tert.butylester,
2,6-Dimethyl-4-(3-methylflavon-8-yl)-1,4-dihydropyridin-3,5-dicarbonsäuremethylcyclohexylester,
2,6-Dimethyl-4-(3-methylflavon-8-yl)-1,4-dihydropyridin-3,5-dicarbonsäuremethylphenylester,
2,6-Dimethyl-4-(3-methylflavon-8-yl)-1,4-dihydropyridin-3,5-dicarbonsäuremethyl-2-ethylsulfonylethylester.

**4.** Verfahren zur Herstellung einer Verbindung der Formel (I) nach Anspruch 1, dadurch gekennzeichnet, dass man

a) ein Aldehyd der Formel

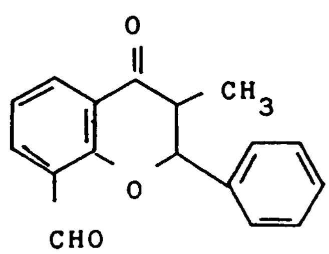

(II)

(nachstehend bezeichnet mit (II)) mit einem Ketoester der Formel

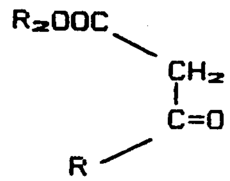

(III)

und mit einem Enamin der Formel

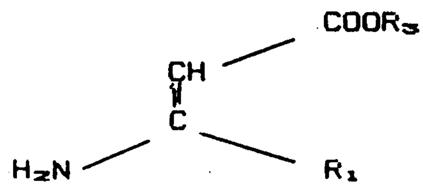

(IV)

worin R, R1, R2 und R3 die oben angegebenen Bedeutungen haben und R, R1 nach ublichen Methoden in Formylalkyl, Hydroxyalkyl oder Cyanalkyl überführt werden können, umsetzt, oder
b) (II) mit einer Verbindung der Formel

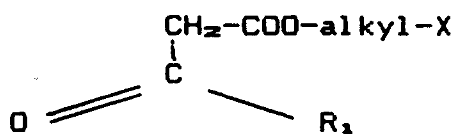

(V)

worin R1 die unter a) angegebene Bedeutung hat und X fur ein Halogenatom steht, umsetzt, und das so erhaltene Arylidenderivat mit einem Enamin der Formel

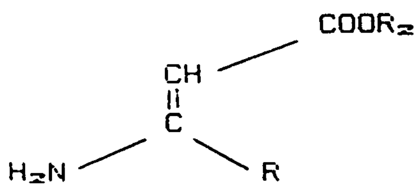

(VI)

worin R1 und R2 die oben angegebenen Bedeutungen haben, behandelt wird, und schliesslich ein Amin der Formel R4R5NH gebenenfalls addiert wird, oder
c) das Aldehyd (II) mit einem Ketoester der Formel (III) umsetzt, und dann zu dem erhaltenen Arylidenderivat ein Enamin der Formel

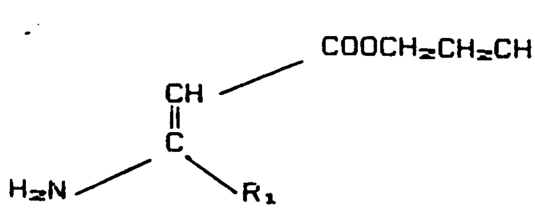

(VII)

in der R, R1 und R2 die oben angegebenen Bedeutungen haben oder es gielt was unter a) geschrieben ist, zugegeben, und der so erhaltene Cyanethylester zu der Säure der Formel

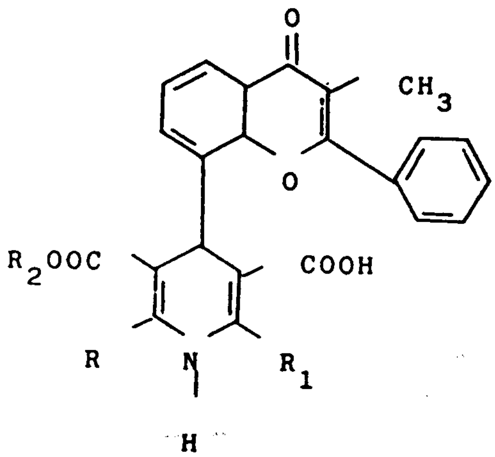

(VIII)

hydrolisiert, die nach bekannten Verfahren mit einem Derivat der Formel R3OH oder R3X, worin X ein Halogenatom ist und R3 die oben angegebene Bedeutung hat, verestert wird.

5. Verfahren nach Anspruch 4, dadurch gekennzeichnet, dass Reaktionsweg a) in einem niedrigen Alkohol, vozugsweise Ethanol, 2 bis 5 Stunden lang und an der Rückflusstemperatur des verwendeten Alkohols durchgeführt wird.

6. Verfahren nach Anspruch 4, dadurch gekennzeichnet, dass die erste Stufe des Reaktionswegs a) in einem chlorierten Kohlenwasserstoff, vorzugszweise Chloroform, der Zusatz von (VI) in einem niedrigen Alkohol, vorzugsweise Isopropanol, und die optionale Umsetzung mit dem Amin R4R5NH in Dimethylformamid bei hoch Temperatur, vorzugsweise bei 100 ° C, durchgeführt wird.

7. Verfahren nach Anspruch 4, dadurch kekennzeichnet, dass die erste Stufe des Reaktionswegs a) in einem chlorierten Kohlenwasserstoff, vorzugsweise Chloroform, dann der Zusatz des Enamins in Alkohol, vorzugszeise Isopropanol, die Hydrolyse der Säure in Dimethoxyethan und die Veresterung in Dimethylformamid durchgeführt wird.

8. Verfahren nach Anspruch 4-7, dadurch gekennzeichnet, dass die Umwandlung von R und R1 zu der Formylgruppe durch Cyclisierung einer Verbindung der Formel (VII) oder (VIII), worin R oder R1 geminales Dialkoxyalkyl ist, und dann durch nachfolgende Hydrolyse mit Chlorwasserstoff in Aceton des erhaltenen 1,4-Dihydropyridinerings mit der oben angegebenen Dialkoxyalkylgruppe in R oder R1, durchgeführt wird.

9. Verfahren nach Ansprüchen 4-8, dadurch gekennzeichnet, dass die Umwandlung von R oder R1 zu der Hydroxyalkylgruppe durch Reduktion der entsprechenden Formylalkylgruppe mit Natriumborhydrid in Alkohol, vorzugsweise Ethanol, durchgeführt wird.

10. Verfahren nach Ansprüchen 4-8, dadurch gekennzeichnet, dass die Umwandlung von R oder R1 zu einer Cyanalkylgruppe durch Umsetzung der entsprechenden Formylalkylgruppe mit Hydroxylamin und Essigsäureanhydrid durchgeführt wird.

11. Pharmazeutische Zubereitungen mit Calciumantagonistischer Wirkung und/oder mit relaxierender Wirkung an der glatten Muskulatur (insbesondere an der Harnblase) bestehend aus einer Verbindung der Formel (I) nach Anspruch 1 und einem geeigneten Träger und Verdünnungsmittel.

12. Pharmazeutische Zubereitungen nach Anspruch 11 für die orale oder parenterale Verabreichung, die sind in der gewöhnlichen Dosen für diese Art von Arzneimitteln anzuwenden.